# EUROPÄISCHE PATENTSCHRIFT

(11) **EP 1 924 200 B1**
(45) Veröffentlichungstag und Bekanntmachung des Hinweises auf die Patenterteilung: **09.02.2011**
(21) Anmeldenummer: 06777190.7
(22) Anmeldetag: 14.09.2006
(51) Int. Cl.: A61B 5/15, F03G 7/06

(54) **VORRICHTUNG ZUM GEWINNEN VON KÖRPERFLÜSSIGKEIT FÜR ANALYSEZWECKE**
DEVICE FOR OBTAINING BODILY FLUIDS FOR ANALYSIS PURPOSES
DISPOSITIF PERMETTANT DE RECUEILLIR DES VOLUMES HUMORAUX A DES FINS D'ANALYSE

(30) Priorität: 15.09.2005 EP 05020062
(43) Veröffentlichungstag der Anmeldung: 28.05.2008
(73) Patentinhaber: F.Hoffmann-La Roche AG, 4070 Basel (CH); Roche Diagnostics GmbH, 68305 Mannheim (DE)
(72) Erfinder: KORNER, Stephan, CH-6330 Cham (CH); PARTEL, Robert, CH-6340 Baar/ZG (CH); CALASSO, Irio Giuseppe, CH-6415 Arth (CH); DECKER, Désirée, CH-6243 Egolzwil (CH)
(74) Vertreter: Pfiz, Thomas
(86) Internationale Anmeldenummer: PCT/EP2006/008952
(87) Internationale Veröffentlichungsnummer: WO 2007/031310

(56) Entgegenhaltungen:
- WO-A-02/068820
- WO-A-03/088835
- WO-A-03/095798
- US-A1- 2004 098 009
- US-A1- 2005 023 086
- US-A1- 2005 165 415

## Beschreibung

Die Erfindung betrifft eine Vorrichtung zum Gewinnen von Körperflüssigkeit für Analysezwecke mit einem in ein Körperteil einstechbaren Stechelement und einem Antrieb für eine vorwärts und zurück verlaufende Stechbewegung des Stechelements.

Solche Entnahmesysteme für kleine Körperflüssigkeitsmengen dienen vor allem Diabetikern zur täglich mehrfach durchgeführten Blutzucker-Selbstkontrolle im Rahmen einer Insulinbehandlung. Für die Gewinnung von Kapillarblut ist es erforderlich, eine Hautöffnung durch einen Einstich zu erzeugen, wobei der Einstichschmerz und die Narbenbildung möglichst weitgehend reduziert werden sollen und zugleich eine hygienische Verfahrensweise gewährleistet sein soll. Damit auch von Laien die erforderlichen Schritte einfach und schnell vorgenommen werden können, ist es wünschenswert, einen weitgehend automatischen Messablauf in einem kompakten Handgerät zu realisieren. Die herkömmlichen Stechapparate sehen federgetriebene Stechantriebe vor, die sich durch eine schnelle Entnahmegeschwindigkeit der gespeicherten Energie auszeichnen. Wenn allerdings die Stechbewegung auch das Sammeln von Blut in so genannten integrierten Systemen umfassen soll, ist eine komplexe Bewegungssteuerung erforderlich, um insbesondere bei kleinen Entnahmemengen eine hohe Verfahrenssicherheit zu gewährleisten.

Das Dokument US-A 2004/0098009 offenbart einen aus zwei gesonderten Einheiten aufgebauten Stechantrieb mit einer mechanischen (federgetriebenen) Vorschubeinheit und einer elektrisch arbeitenden Rückholeinheit. Für letztere wird allgemein die Verwendung eines dort so genannten "Nanomuskel", d. h. einer Formgedächtnislegierung vorgeschlagen, wobei jedoch nicht auf spezifische Ausführungsformen eingegangen wird. In dem angeführten Beispiel wird der Aktuator folglich weder für eine schnelle Einstichbewegung noch für eine kombinierte Hin- und Rückbewegung eines Stechelements verwendet.

Das Dokument WO 02/068820 beschreibt SMA-Aktuatoren mit verbesserter Temperaturkontrolle und befasst sich insbesondere mit einer Verkürzung der Abkühlzeiten, die durch die spezielle Ausgestaltung eines SMA-Drahts in Verbindung mit Platten als Wärmesenke. Das Dokument bezieht sich jedoch nicht auf die Integration eines SMA-Aktuators in Stechhilfen und befasst sich folglich auch nicht mit den hierdurch entstehenden technischen Problemen. Bei den beschriebenen Abkühlzeiten wäre die Verwendung in einer Stechhilfe auch nicht akzeptabel und an die Einstichbewegung würde nicht unmittelbar eine Rückführbewegung anschließen, so dass das Stechorgan schmerzhaft in der Haut verbleiben würde. US 20005/023086 und WO 03/095798 offenbaren ähnliche SMA-Aktuatoren.

Ausgehend hiervon liegt der Erfindung die Aufgabe zugrunde, die im. Stand der Technik aufgetretenen Nachteile zu vermeiden und mit einfachen Mitteln eine optimierte Gewinnung der Körperflüssigkeit zu ermöglichen.

Zur Lösung dieser Aufgabe wird die in Anspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen hochdynamischen Antrieb mit hoher Energiedichte für eine kontrollierte Hin- und Herbewegung bereitzustellen. Insbesondere soll eine hohe Geschwindigkeit beim Eindringen in die Haut und eine schnelle und unmittelbare Umsetzung in eine Rückholbewegung erreicht werden. Dementsprechend wird erfindungsgemäß vorgeschlagen, dass der Antrieb mindestens einen Aktuatordraht auf Basis von Formgedächtnislegierungen zur Steuerung der Stechbewegung über eine Veränderung der Drahtlänge aufweist. Solche SMA-Aktuatoren zeichnen sich durch Miniaturisierbarkeit und direkte Erzeugung von Linearbewegungen aus. Während mit üblichen stapelförmig gepackten SMA-Aktuatoren eine Hin- sowie anschließende Rückbewegung einer Stecheinheit nicht praxisgerecht realisiert werden kann, hat sich überraschend erwiesen, dass die Energieentnahmegeschwindigkeiten und Bewegungsamplituden von SMA-Drahtaktuatoren hinreichend sind, um eine hochdynamische Bewegung für eine Stech- und Sammelfunktion zu verwirklichen. Durch einfache thermische Beeinflussung kann eine lineare Stechbewegung erzeugt werden, wobei die geringe Wärmemenge, die zur Aufheizung des dünnen Drahtmaterials erforderlich ist, einen schnellen Bewegungsablauf erlaubt.

Eine vorteilhafte Ausführung sieht vor, dass der Aktuatordraht durch wärmeaktivierte Kontraktion ein Vorschubmittel für die Vorwärtsbewegung des Stechelements bildet. Dabei können gerade durch die Drahtausführung die kurzen Zeitspannen und hinreichenden Einstechtiefen realisiert werden.

Besonders bevorzugt sind Ausführungen, bei denen der Antrieb einen zwischen dem Aktuatordraht und dem Stechelement angeordneten Bewegungswandler zum Umsetzen einer Längenveränderung des Aktuatordrahts in die Stechbewegung aufweist. Auf diese Weise lassen sich komplexe Bewegungsprofile darstellen, wie sie für eine kombinierte Stech- und Sammelfunktion vorteilhaft sind.

Vorteilhafterweise umfasst der Antrieb eine Heizeinheit zum Aufheizen des Aktuatordrahts auf eine die Kontraktion der Formgedächtnislegierung bewirkende Heiztemperatur. Dies lässt sich auf einfache Weise dadurch realisieren, dass an dem Aktuatordraht eine einen elektrischen Stromstoß erzeugende Stromquelle, insbesondere ein Kondensator über einen Auslöseschalter angeschlossen ist. Grundsätzlich sind aber auch andere Heizmethoden denkbar, beispielsweise über Heißluft, Wärmestrahlung und Wärmeleitung.

Zum Einstellen der Stechtiefe und/oder Sammeltiefe des Stechelements kann es von Vorteil sein, wenn der Aktuatordraht vorzugsweise durch verschiebbare elektrische Kontaktstellen über eine veränderliche Länge aufheizbar ist. Vorteilhaft ist es auch, wenn der Aktuatordraht zum Einstellen der Stechtiefe und/oder Sammeltiefe des Stechelements durch gezieltes Aufheizen auf eine Gefügeumformungstemperatur einer Teilkontraktion unterworfen ist.

Eine Art von Hybridantrieb lässt sich durch ein bei der Vorwärtsbewegung des Stechelements vorspannbares Rückstellmittel, insbesondere eine Rückstellfeder zur Rückbewegung des Stechelements realisieren. Allgemein wird somit die Drahtkontraktion in eine Vorwärts- und eine unmittelbar anschlieβende Rückbewegung des Stechelements umgesetzt.

Eine besonders bevorzugte Ausführung sieht vor, dass der oder ein Aktuatordraht die Rückbewegung des Stechelements bremst und/oder dämpft. Dadurch lässt sich die Blutaufnahme durch langsames Zurückziehen des Stechelements günstig beeinflussen.

Eine weitere vorteilhafte Variante sieht zwei wechselweise kontrahierbare Aktuatordrähte zur alternierenden Steuerung der Stechbewegungen in aufeinander folgenden Zyklen vor.

Grundsätzlich ist für eine solche Ausgestaltung ein Drahtmaterial nicht zwingend, so dass ein Erfindungsaspekt auch darin liegt, dass der Antrieb zwei wechselweise kontrahierbare Aktuatoren auf Basis von Formgedächtnislegierungen zur alternierenden Steuerung der Stechbewegungen in aufeinander folgenden Zyklen aufweist.

Eine weitere Verbesserung sieht vor, dass ein Aktuatordraht unter Kontraktion die Vorwärtsbewegung des Stechelements treibt und der andere Aktuatordraht unter Expansion die Rückwärtsbewegung bremst und/oder dämpft.

Für eine bremsende bzw. dämpfende Wirkung ist es möglich, dass der als Bremsmittel wirkende Aktuatordraht durch Vorheizen auf eine Gefügeumformungstemperatur der Formgedächtnislegierung gezielt verkürzt und unter Abkühlung entsprechend verlängerbar ist.

Eine weitere vorteilhafte Ausgestaltung besteht darin, dass der die Rückbewegung bremsende bzw. dämpfende Aktuatordraht abschnittsweise vorgeheizt ist.

Denkbar ist es auch, dass der Aktuatordraht durch zugspannungsinduzierte Phasenumwandlung der Formgedächtnislegierung als Bremsmittel und/oder Dämpfungsmittel wirkt.

Für eine hohe Dynamik auch bei der Rückbewegung ist es von besonderem Vorteil, wenn der Antrieb einen in der Achse der Stechbewegung streckbaren Kniehebelmechanismus oder eine entsprechend streckbare Blattfeder umfasst. Dabei kann ein Bewegungszyklus dadurch realisiert werden, dass der Kniehebelmechanismus oder die Blattfeder durch den Aktuatordraht in eine Streckstellung und durch ein in der Streckstellung vorgespanntes Rückstellelement in eine Beugestellung bringbar ist.

Zur Einstellung der Stechtiefe die Lagerstelle eines von dem Stechelement abgewandten Schwenklagers des Kniehebelmechanismus oder der Blattfeder in der Stechachse verstellbar sein.

Eine Verlangsamung der Rückbewegung des Stechelements lässt sich auch durch ein insbesondere als Kolben-Zylinder-Einheit ausgebildetes Dämpfungselement realisieren.

Für ein schnelles Aufheizen ist es vorteilhaft, wenn der Durchmesser des Aktuatordrahts kleiner als 1 mm, vorzugsweise kleiner als 0,5 mm ist.

Um den Einfluss einer schwankenden Umgebungstemperatur zu minimieren, ist es günstig, wenn die insbesondere als Nickel-Titan-Legierung ausgebildete Formgedächtnislegierung eine Umformungstemperatur von mehr als 100°C besitzt.

Zur Erhöhung der wirksamen Drahtlänge in gegebenem Bauraum ist es von Vorteil, wenn der Aktuatordraht mehrere über Umlenkmittel geführte, nebeneinander verlaufende Drahtabschnitte aufweist.

Für eine Verstärkung der Antriebskraft kann es vorteilhaft sein, wenn der Aktuatordraht aus mehreren parallel zueinander verlaufenden Einzeldrähten besteht.

Vorteilhafterweise erfolgt die Vorwärtsbewegung des Stechelements zur Erzeugung eines Hauteinstichs mindestens eine Größenordnung schneller als die Rückbewegung zum Sammeln von Körperflüssigkeit.

Ein weiterer Erfindungsaspekt umfasst eine Entnahmevorrichtung für Körperflüssigkeit, bei welcher der Antrieb einen durch elektro-aktive Polymere (EAP) gebildeten EAP-Aktuator aufweist. Auch dadurch lässt sich ein hochdynamischer Bewegungsablauf mit geeignetem Stech- und Sammelprofil in kompakter Bauweise ohne motorische Mittel realisieren.

Vorteilhafterweise weist der EAP-Aktuator ein zwischen zwei mit Hochspannung beaufschlagbaren Elektroden angeordnetes Elastomerelement auf, wobei eine durch elektrostatische Anziehung der Elektroden bewirkte Verformung des Elastomerelements in die Stechbewegung umsetzbar ist.

Im Folgenden wird die Erfindung anhand der in der Zeichnung schematisch dargestellten Ausführungsbeispiele näher erläutert. Es zeigen
- Fig. 1: eine SMA-getriebene Blutentnahmevorrichtung in einer per- spektivischen Darstellung;
- Fig. 2: verschiedene Positionen bei der Stechbewegung in der Sei- tenansicht der Vorrichtung nach Fig. 1;
- Fig. 3: ein Zeitdiagramm der vorwärts und zurück verlaufenden Stechbewegung;
- Fig. 4: ein Schaltbild einer elektrischen Heizeinheit zur Aktivierung eines Antriebs für die Stechbewegung;
- Fig. 5: ein Diagramm eines SMA-Hysteresezyklus;
- Fig.6: ein typisches Spannungs/Dehnungsdiagramm für SMA- Materialien;
- Fig. 7: eine EAP-getriebene Stech/Sammelvorrichtung in einem schematisch stark vereinfachten Schaubild; und
- Fig. 8 bis 10: weitere Ausführungsbeispiele für SMA-Stechvorrichtungen in schematischer Darstellung.

Die in der Zeichnung dargestellten Stech- bzw. Sammelvorrichtungen zum Gewinnen kleiner Mengen von Blut für Blutzuckertests umfassen ein in ein nicht gezeigtes Körperteil (beispielsweise eine Fingerkuppe) einstechbares Stechelement 10, einen Antrieb 12 für eine vor und zurück verlaufende Stechbewegung des Stechelements 10 und eine Aufnahme 14 für den Antrieb und die Linearführung des Stechelements.

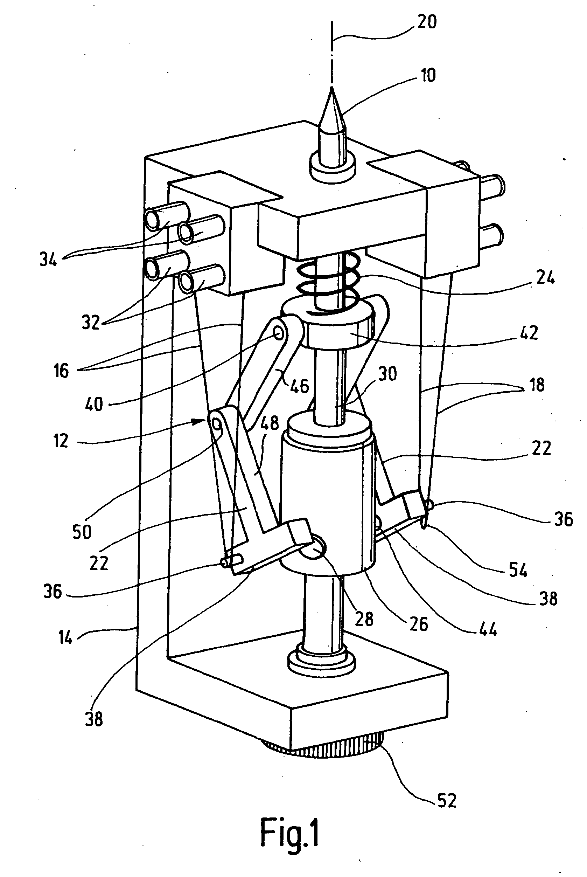

Fig. 1 zeigt einen Testaufbau für einen mittels zwei Aktuatordrähten 16, 18 auf Basis von Formgedächtnislegierungen (Shape Memory Alloys, kurz: SMA) aktuierbaren Antrieb 12. Solche SMA-Aktuatoren erfahren bei Erreichen einer bestimmten Umwandlungstemperatur eine Formänderung, speziell eine Verkürzung der Drahtlänge, die sich zur Erzeugung einer Stech- bzw. Sammelbewegung ausnutzen lässt. Zu diesem Zweck umfasst der Antrieb 12 einen in der Achse 20 der Stechbewegung streckbaren Kniehebelmechanismus 22 als Bewegungswandler. Dieser ist durch die wechselweise kontrahierbaren Aktuatordrähte 16, 18 in aufeinander folgenden Stechzyklen in die Streckstellung bringbar, wobei eine dabei gespannte Rückstellfeder 24 jeweils wieder für die Rückstellung in die Beugestellung sorgt. Optional kann hier ein Dämpfungszylinder 26 eine zusätzliche Dämpfung der Rückbewegung bewirken. Der Dämpfungszylinder 26 besitzt ein Ventil 28, welches die Dämpfungsrichtung steuert, so dass eine ungedämpfte schnelle Vorwärtsbewegung und eine gedämpfte langsamere Rückbewegung der in dem Zylinder geführten Kolbestange 30 erreicht wird.

Die Aktuatordrähte 16, 18 sind an ihren Enden an Spannstücken 32 aufnahmeseitig eingespannt und dabei über Anschlussbuchsen 34 elektrisch kontaktierbar. Ein Mittelstück der Aktuatordrähte 16, 18 ist über einen Umlenkzapfen 36 an jeweils einem Schenkelende eines T-Auslegers 38 des Kniehebelmechanismus 22 geführt, so dass durch zwei nebeneinander verlaufende Drahtabschnitte eine große Drahtlänge erreicht wird. Um eine rasche elektrische Aufheizung mittels Joule-Effekt zu ermöglichen, kann der Drahtdurchmesser kleiner als 0,5 mm gewählt werden. Zur Verringerung des Einflusses schwankender Umgebungstemperaturen kann eine Nickel-Titan-Legierung, insbesondere Nickel-Titan-Hafnium oder Nickel-Titan-Zirkonium mit einer Umformungstemperatur von mehr als 100°C als Drahtmaterial eingesetzt werden.

Der Kniehebelmechanismus weist zwei parallele Kniehebeltriebe 22 auf, die über ein distales Schwenklager 40 gelenkig an einen Bund 42 der Stange 30 angeschlossen sind und über ein proximales Widerlager 44 bei der Stechbewegung ortsfest abgestützt sind, wobei das die Kniehebel 46, 48 verbindende Kniegelenk 50 frei schwingt. Die Stechtiefe des Stechelements 10 lässt sich mittels einer Stellvorrichtung 52 einstellen, welche die Position des das Widerlager 44 tragenden Zylinders 26 in Richtung der Stechachse 20 festlegt.

Wie bereits erwähnt, sind die Aktuatordrähte 16, 18 für einen wechselseitigen Antrieb vorgesehen, wobei in der in Fig. 1 gezeigten Stellung der Draht 16 durch wärmeaktivierte Kontraktion ein Vorschubmittel für die Vorwärtsbewegung des Stechelements 10 bildet, während der andere Draht 18 unter Expansion die Rückbewegung des Stechelements 10 bremst bzw. dämpft. Hierbei kann der bremsende Draht 18 eine über die Umlenkstelle 36 überstehende Schlaufe 54 aufweisen, so dass der Bremseingriff erst nach einem gewissen Rückhub verzögert erfolgt.

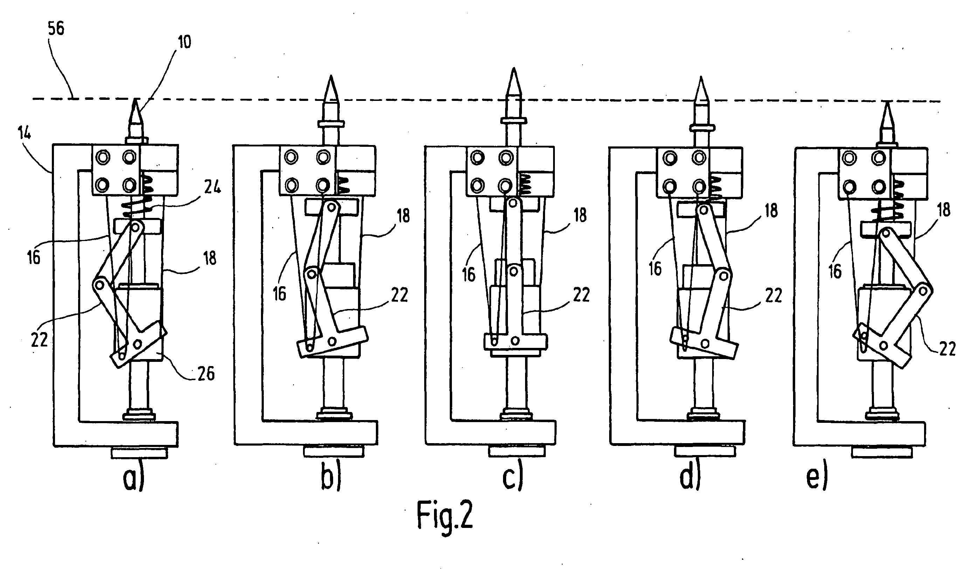

Der Ablauf der Stechbewegung ist in Fig. 2 in verschiedenen Positionen veranschaulicht. In der Ausgangsposition Fig. 2a ist der obere Draht 16 gespannt und der untere Draht 18 relaxiert. Das Stechelement 10 befindet sich mit seiner Spitze in der Nullposition 56 des Stechhubs. Sodann wird der Draht 16 durch einen Stromstoss erhitzt. Bei Erreichen der Umformungstemperatur kontrahiert der Draht 16 und das Stechelement 10 bewegt sich vorwärts (Fig. 2b). Die maximale Vorschublage wird in der Streckstellung des Kniehebelmechanismus 22 erreicht (Fig. 2c), in welcher auch die Rückstellfeder 24 maximal gespannt ist. Sodann verkürzt sich der Draht 16 nicht wesentlich weiter, und der Kniehebelmechanismus schwingt unter der Kraft der Rückstellfeder 24 in die gegenüberliegende Knickstellung Fig. 2d, in der mittels des Dämpfers 26 und/oder des hinteren Aktuatordrahts 18 die weitere Rückbewegung des Stechelements 10 verzögert wird, um so eine ausreichende Sammelzeit für die Blutaufnahme über einen in dem Stechelement integrierten, nicht eigens gezeigten Sammelkanal zu gewährleisten. Auf diese Weise kann ein schneller schmerzarmer Einstich mit einer mindestens eine Größenordnung langsameren Sammelperiode in einem Bewegungsablauf kombiniert werden, bis schließlich die Endposition Fig. 2e erreicht wird. Dabei kann der Draht 16 langsam auskühlen, während der Draht 18 für den nächsten Stechzyklus gespannt ist, ohne dass ein Benutzereingriff erforderlich wäre.

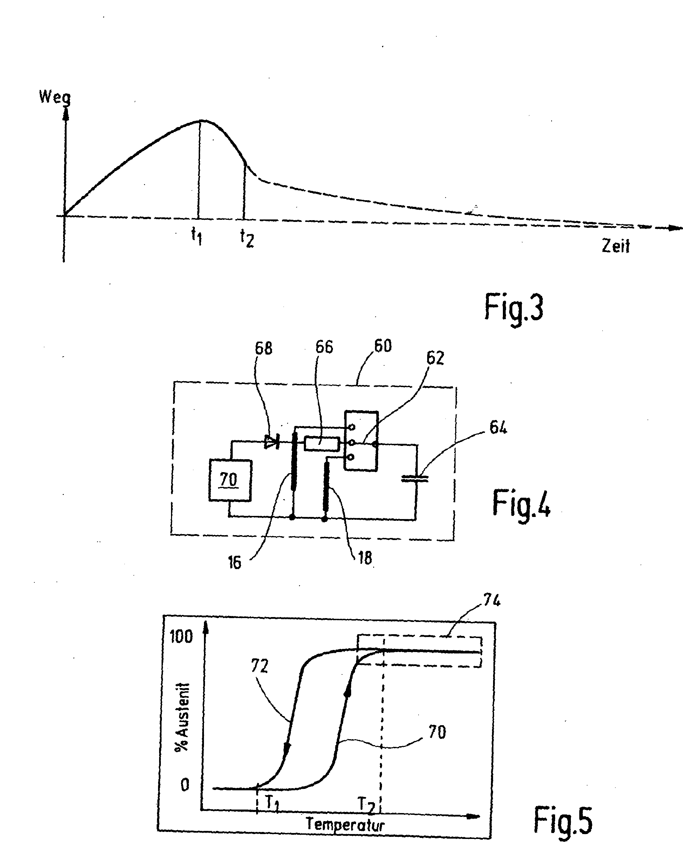

Fig. 3 veranschaulicht nochmals den Ablauf der Stechbewegung in einem Weg-Zeit-Diagramm. Die schnelle Einstichphase verläuft bis zu einer Zeit t1, in der die maximale Stechtiefe erreicht ist. Beispielsweise kann t1 wenige Millisekunden und die Stechtiefe einige Millimeter betragen. Anschließend erfolgt eine anfänglich rasche Rückbewegung bis zum Zeitpunkt t2, ab welchem in einer geeigneten zurückgezogenen Sammeltiefe die Körperflüssigkeit langsam aufgenommen wird. Diese Sammelphase kann im Sekundenbereich liegen, so dass auch allein durch Kapillarwirkung eine ausreichende Flüssigkeitsmenge gewonnen wird.

Damit der Kniehebelmechanismus 22 hin und her schwingen kann, muss abwechselnd der eine, dann der andere Draht 16, 18 aufgeheizt werden. Zu diesem Zweck weist der Antrieb eine elektrische Heizeinheit 60 auf, die gemäß dem Schaltungsbeispiel Fig. 4 ausgelegt sein kann. In der gezeigten Nullstellung des Wechselschalters 62 wird der Kondensator 64 in Serie mit dem Widerstand 66 und der Diode 68 aus der Spannungsquelle 70, speziell einer Batterie aufgeladen. In der oberen Schaltstellung des Schalters 62 kann dann der Kondensator 64 über den Draht 16 entladen werden, wobei durch den erzeugten Stromstoss aufgrund der geringen Wärmekapazität des dünnen Drahts das Aufheizen in sehr kurzer Zeitdauer möglich ist. Anschlieβend wird in der Nullstellung der Kondensator 64 wieder aufgeladen, so dass in der unteren Schaltstellung nunmehr der andere Draht 18 aktiviert werden kann.

Um die langsame Rückbewegung während der Sammelphase zu steuern, kann der jeweils als Bremsmittel eingesetzte zweite SMA-Draht 16, 18 bis zu einer gewissen Vorspannlänge vorgeheizt werden. Die durch die Rückstellfeder 24 getriebene Rückbewegung wird dann vorzeitig abgebremst und unter Abkühlung des betreffenden Drahtes zeitlich verzögert. Dies kann durch kontrolliertes Erhitzen der Formgedächtnislegierung in das Gefügeumformungs-Temperaturband oder durch Teilerhitzen nur eines Teils der Drahtlänge erreicht werden.

Zur näheren Erläuterung des kontrollierten Erhitzens ist in Fig. 5 ein typischer Hysteresezyklus für die temperaturabhängige Umformung einer Formgedächtnislegierung gezeigt. Beim Aufheizen vollzieht sich entsprechend der Kurve 70 ein Phasenübergang in der metallurgischen Struktur von der martensitischen in die austenitische Form. Der umgekehrte Übergang beim Abkühlen zeichnet sich entsprechend der Kurve 72 durch ein hysteretisches Verhalten der Struktur-Temperatur-Beziehung aus. Durch die Strukturänderung tritt in der Austenitphase eine Spannungserholung und damit einhergehend eine Kontraktion in eine "erinnerte" Form auf. Daraus folgt, dass innerhalb des Umformungstemperaturintervalls zwischen T1 und T2 die Drahtlänge gezielt verkürzt werden kann, um durch entsprechende Expansion bei der Abkühlung die Rückbewegung des Stechelements zu bremsen. Problematisch ist hierbei der Einfluss der Umgebungstemperatur, der durch geeignete Wärmeisolation des Drahts oder durch Wahl eines Materials mit hoher Umwandlungstemperatur minimiert werden kann.

Um solche Probleme zu umgehen, ist es auch denkbar, den jeweils bremsenden bzw. dämpfenden Aktuatordraht nur segmentweise zu erhitzen. Dies lässt sich durch entsprechende elektrische Abgriffe an dem Draht realisieren, die gegebenenfalls verschiebbar angebracht sein können. Das oder die erhitzten Segmente werden dabei in einer Ein/Aus-Aktuierung betrieben, d.h. deutlich über das Umformungstemperaturintervall hinaus erhitzt. Somit wird der Draht immer um dieselbe Länge kontrahiert, welche dann beim Abkühlen als definierter Bremsweg zur Verfügung steht.

In dem in Fig. 5 gestrichelt umrahmten Bereich 74, der sich an das Umformungstemperaturintervall anschließt, zeigen SMA-Materialien unter Deformation ein superelastisches Verhalten. Dieser Effekt wird durch spannungsinduzierte Martensitbildung (Englisch: "stress-induced martensite formation") verursacht. Weil der Martensitanteil oberhalb seiner normalen Temperatur gebildet wurde, wandelt er sich unmittelbar in undeformierten Austenit um, sobald die äußere Belastung wegfällt. Auch diese Materialeigenschaft kann dazu genutzt werden, die Rückbewegung des Stechelements 10 ohne große Vibrationen abzudämpfen. Das Drahtmaterial muss dafür lediglich vor Einsetzen der mechanischen Belastung in den Bereich 74 erhitzt werden, damit diese Dämpfung dann einsetzt.

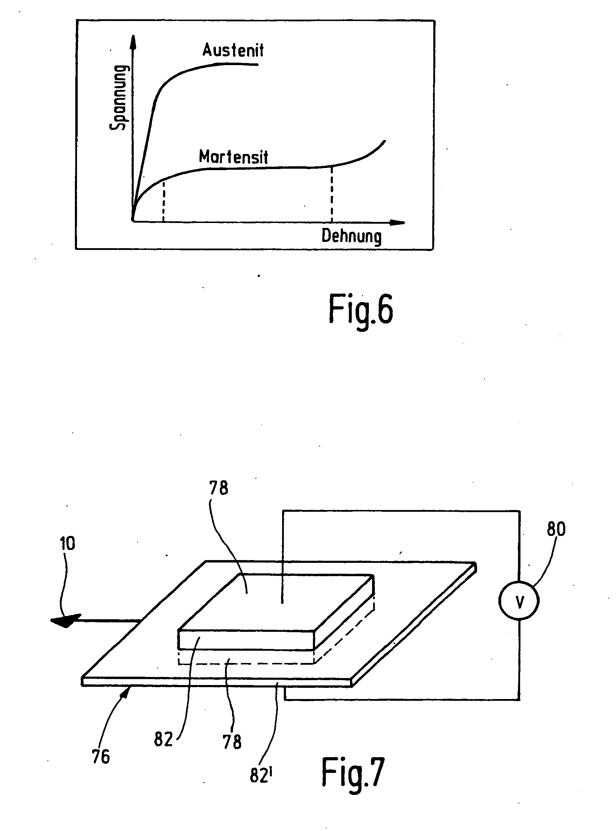

Eine weitere Dämpfungseigenschaft von SMA-Material tritt in der kühleren Martensit-Phase auf, wenngleich weniger effektiv als die spannungsinduzierte Martensitbildung. Entsprechend Fig. 6 besitzt die Martensit-Phase in dem gestrichelt markierten Dehnungsintervall eine quasi-ideale Dämpfung, die ebenfalls für eine Verlangsamung der Rückbewegung des Stechelements 10 nutzbar ist.

Fig. 7 veranschaulicht die prinzipielle Funktionsweise einer Ausführungsform einer Stech- und Sammelvorrichtung basierend auf einem durch elektro-aktive Polymere (EAP) gebildeten EAP-Aktuator 76. Dieser weist als dielektrischer EAP-Aktuator zwei einander gegenüberliegende Flächenelektroden 78 auf, die über eine geeignete Spannungsquelle 80 mit Hochspannung beaufschlagbar sind. Zwischen den Elektroden 78 befindet sich ein Elastomerblock 82. Das Aktuatorprinzip beruht auf einer hochdynamischen Verformung dieses Elastormerblocks 82 beim Aufschalten der Hochspannung auf die Elektroden 78. Diese ziehen sich dann aufgrund elektrostatischer Wechselwirkung an, wodurch das inkompressible Elastomermaterial unter Querverformung eine Ausdehnung in die flache Konfiguration 82' erfährt. Voraussetzung dafür sind elastische Elektroden 78, welche die Flächenausdehnung aufnehmen können. Die Querverformung kann für eine lineare Stechbewegung des Stechelements 10 genutzt werden. Durch eine geeignete Geometrie des Elastomerelements und der Kopplung des Stechelements kann dabei ein großer Hub in der Stechachse erreicht werden. Eine Kapazitätsmessung über die Elektroden 78 erlaubt eine Feedback-Regelung der erzeugten Bewegung über einen auf die Hochspannungsquelle 80 einwirkenden geschlossenen Regelkreis.

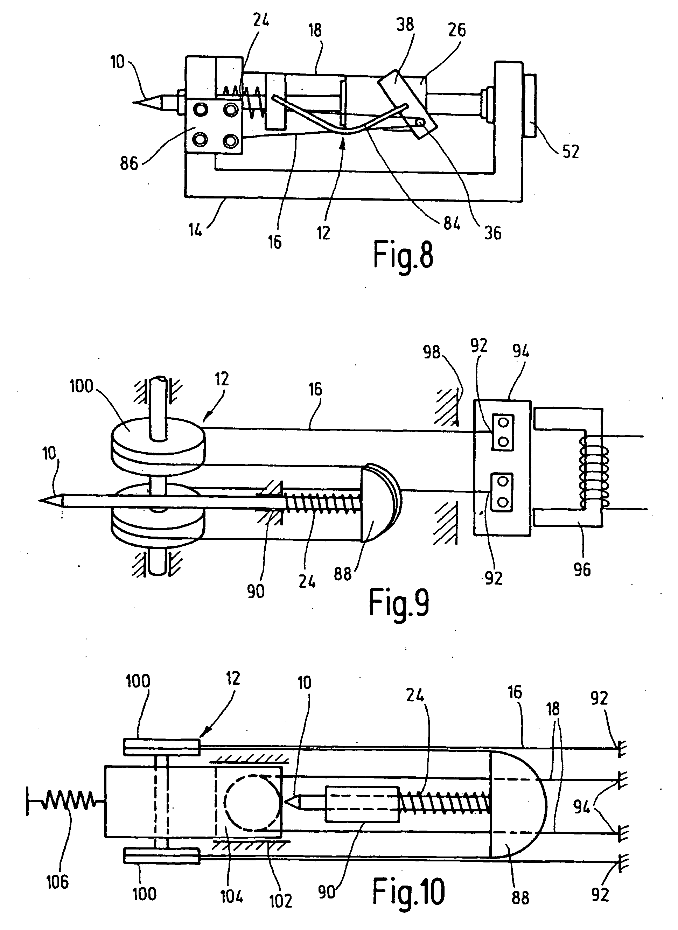

Fig. 8 zeigt ein weiteres Ausführungsbeispiel einer durch SMA-Drähte 16, 18 getriebenen Stechvorrichtung ähnlich der Ausführung nach Fig. 1, wobei gleiche Teile mit gleichen Bezugszeichen versehen sind. Als Kniehebelmechanismus 22 wird hier eine Blattfeder 84 eingesetzt. Diese ist an den Federenden fest eingespannt, wobei der Ausleger 38 um seine Mittelachse schwenkbeweglich gelagert ist, so dass unter Umklappen der Blattfeder 84 eine hin- und hergehende Stechbewegung entsprechend Fig. 2 möglich ist. Hierbei kann auf eine Kniegelenkachse verzichtet werden. Auf diese Weise kann die Baugröße weiter verringert werden, und es wird ein mechanisches Gelenkspiel vermieden. Vorteilhafterweise ist bei einer Verstellung der Stechtiefe mittels Stellvorrichtung 52 der Anschlussblock 86 für die SMA-Drähte 16, 18 korrespondierend verstellbar, so dass der Abstand zu dem Umlenkzapfen 36 erhalten bleibt.

Fig. 9 zeigt eine Alternative zu einem Kniehebelmechanismus mit nur einem SMA-Draht 16, welcher das Stechteil 10 über eine Halterung 88 bewegt. Zu diesem Zweck ist die Halterung 88 mit dem Stechteil 10 in einer gerätefesten Linearführung 90 über eine Rückstellfeder 24 abgestützt. Die beiden Enden 92 des Drahtes 16 sind an einer ferromagnetischen Platte 94 angeschlossen, die sich mittels Elektromagnet 96 von einem Anschlag 98 abheben lässt. Der SMA-Draht 16 ist zwischen seinen Enden 92 über eine drehbare Doppel-Umlenkrolle 100 und das Kopfstück der Halterung 88 mehrfach umgelenkt. Bei Einschalten des Magnets 96 wird die Platte 94 angezogen, so dass die Nadel 10 nach vorne gerückt wird. Beim anschließenden Verkürzen des Drahts 16 durch einen Heizstromstoss schnellt die Nadel 10 weiter vor. Am Umkehrpunkt wird der Magnet 96 abgeschaltet, und die Platte 94 stößt unter Expansion der Rückstellfeder 24 gegen den Anschlag 98, wodurch ein schnelles Zurückziehen der Nadel 10 bewirkt wird. Die Rückziehgeschwindigkeit ist dabei viel höher als bei einer bloßen Drahtabkühlung. Um die Nadel 10 noch eine Weile in der Haut verweilen zu lassen, kann der Draht 16 nachgewärmt werden. Danach lässt man ihn abkühlen. Dieser Aufbau besitzt einige apparative Vorteile. So kann die Nadel 10 im Inneren eines Geräts verweilen und erst durch den Vorhub nach der Magnetbetätigung aktiviert werden. Die Rückstellfeder 24 wird erst dadurch vorgespannt und ermüdet daher weniger. Weiterhin kann die Stechtiefe relativ einfach durch eine Abstandsveränderung des Magnets 96 zu dem Anschlag 98 eingestellt werden. Die Baulänge der Vorrichtung lässt sich aufgrund der Drahtumlenkung 100 verkürzen. Außerdem findet beim Ausschalten des Magnets 96 eine Bewegungsdämpfung durch den noch warmen SMA-Draht 16 statt.

In der Ausführungsform nach Fig. 10 sind zwei SMA-Drahtaktuatoren 16, 18 vorgesehen, die mit ihren Enden 92 bzw. 94 gerätefest eingespannt sind. Der längere Draht 16 treibt die Einstechbewegung, wobei Umlenkrollen 100 und eine Nadelhalterung 88 entsprechend Fig. 9 als Umlenkstellen vorgesehen sind. Der kürzere Draht 18 dient dazu, die Nadel schnell wieder aus dem Körperteil herauszuziehen. Hierfür ist in der Linearführung 102 ein Umlenkschlitten 104 gelagert, welcher bei einer Verkürzung des Drahts 18 die Zugfeder 106 expandiert und dabei die Umlenkrollen 100 entgegen der Stechrichtung zurückfährt. Demzufolge wird dann die Nadelhalterung 88 unter der Kraft der Rückstellfeder 24 um den doppelten Rückstellhub zurückbewegt. Damit die beschrieben Funktionalität des Antriebs 12 gewährleistet ist, muss die die Federkonstante der Zugfeder 106 viel größer sein als die Federkonstante der Rückstellfeder 24. Dadurch bewegt sich beim Verkürzen des Drahts 16 der Umlenkschlitten 104 im Verhältnis zur Halterung 88 nur wenig. Für die Stechbewegung wird zunächst der längere Draht 16 durch einen Stromstoss aus einem Kondensator erwärmt. Sobald dieser Draht 16 sich zusammengezogen hat, wird auch der zweite Draht 18 strominduziert erwärmt. Nach einer gewissen Rückziehstrecke kommt es zu einer Stabilisierung der Nadel 10, weil die beiden Drähte 16, 18 entgegengesetzt wirken. Eine Feineinstellung des Bewegungsprofils ist einfach möglich durch Anpassen der Zugfeder 106 und durch geeignete Wahl der Länge des Drahts 18.

## Patentansprüche

1. Vorrichtung zum Gewinnen von Körperflüssigkeit für Analysezwecke mit einem in ein Körperteil einstechbaren Stechelement (10) und einem Antrieb (12) für eine vorwärts und zurück verlaufende Stechbewegung des Stechelements (10), **dadurch gekennzeichnet, dass** der Antrieb (12) mindestens einen Aktuatordraht (16,18) auf Basis von Formgedächtnislegierungen zur Steuerung der Stechbewegung über eine Veränderung der Drahtlänge aufweist, wobei die Längenänderung des Aktuatordrahts (16,18) sowohl in eine Vorwärtsbewegung als auch eine direkt daran anschießende Rückbewegung des Stechelements (10) umgesetzt wird.

2. Vorrichtung nach Anspruch 1, **dadurch gekennzeichnet, dass** der Aktuatordraht (16,18) durch wärmeaktivierte Kontraktion ein Vorschubmittel für die Vorwärtsbewegung des Stechelements (10) bildet.

3. Vorrichtung nach Anspruch 1 oder 2, **dadurch gekennzeichnet, dass** der Antrieb (12) einen zwischen dem Aktuatordraht (16,18) und dem Stechelement (10) angeordneten Bewegungswandler (22) zum Umsetzen einer Längenveränderung des Aktuatordrahts (16,18) in die Stechbewegung aufweist.

4. Vorrichtung nach einem der Ansprüche 1 bis 3, **dadurch gekennzeichnet, dass** der Antrieb (12) eine Heizeinheit (60) zum Aufheizen des Aktuatordrahts (16,18) auf eine die Kontraktion der Formgedächtnislegierung bewirkende Heiztemperatur aufweist.

5. Vorrichtung nach einem der Ansprüche 1 bis 4, **dadurch gekennzeichnet, dass** an dem Aktuatordraht (16,18) eine einen elektrischen Stromstoß erzeugende Stromquelle, insbesondere ein Kondensator (64) über einen Auslöseschalter (62) angeschlossen ist.

6. Vorrichtung nach einem der Ansprüche 1 bis 5, **dadurch gekennzeichnet, dass** der Aktuatordraht (16,18) zum Einstellen der Stechtiefe und/oder Sammeltiefe des Stechelements (10) vorzugsweise durch verschiebbare elektrische Kontaktstellen über eine veränderliche Länge aufheizbar ist.

7. Vorrichtung nach einem der Ansprüche 1 bis 6,**dadurch gekennzeichnet, dass** der Aktuatordraht (16,18) zum Einstellen der Stechtiefe und/oder Sammeltiefe des Stechelements (10) durch gezieltes Aufheizen auf eine Umformungstemperatur einer Teilkontraktion unterworfen ist.

8. Vorrichtung nach einem der Ansprüche 1 bis 7, **dadurch gekennzeichnet, dass** der Antrieb (12) ein bei der Vorwärtsbewegung des Stechelements (10) vorspannbares Rückstellmittel (24), insbesondere eine Rückstellfeder zur Rückbewegung des Stechelements (10) aufweist.

9. Vorrichtung nach einem der Ansprüche 1 bis 8, **dadurch gekennzeichnet, dass** der Aktuatordraht (16,18) die Rückbewegung des Stechelements (10) bremst und/oder dämpft.

10. Vorrichtung nach einem der Ansprüche 1 bis 9, **gekennzeichnet durch** zwei wechselweise kontrahierbare Aktuatordrähte (16,18) zur alternierenden Steuerung der Stechbewegungen in aufeinander folgenden Zyklen.

11. Vorrichtung nach Anspruch 10, **dadurch gekennzeichnet, dass** ein Aktuatordraht (16) unter Kontraktion die Vorwärtsbewegung des Stechelements (10) treibt und der andere Aktuatordraht (18) unter Expansion die Rückwärtsbewegung bremst und/oder dämpft.

12. Vorrichtung nach einem der Ansprüche 9 bis 11, **dadurch gekennzeichnet, dass** der als Bremsmittel wirkende Aktuatordraht (16,18) durch Vorheizen auf eine Gefügeumformungstemperatur der Formgedächtnislegierung gezielt verkürzt und unter Abkühlung entsprechend verlängerbar ist.

13. Vorrichtung nach einem der Ansprüche 9 bis 12, **dadurch gekennzeichnet, dass** der die Rückbewegung bremsende bzw. dämpfende Aktuatordraht (16,18) abschnittsweise vorgeheizt ist.

14. Vorrichtung nach einem der Ansprüche 9 bis 13, **dadurch gekennzeichnet, dass** der Aktuatordraht (16,18) durch zugspannungsinduzierte Phasenumwandlung der Formgedächtnislegierung als Bremsmittel und/oder Dämpfungsmittel wirkt.

15. Vorrichtung nach einem der Ansprüche 1 bis 14, **dadurch gekennzeichnet, dass** der Antrieb (12) einen in der Achse der Stechbewegung streckbaren Kniehebelmechanismus (22) oder eine entsprechend streckbare Blattfeder (84) umfasst.

16. Vorrichtung nach Anspruch 15, **dadurch gekennzeichnet, dass** der Kniehebelmechanismus (22) oder die Blattfeder (84) durch den Aktuatordraht (16,18) in eine Streckstellung und durch ein in der Streckstellung vorgespanntes Rückstellelement (24) in eine Beugestellung bringbar ist.

17. Vorrichtung nach Anspruch 15 oder 16, **dadurch gekennzeichnet, dass** die Lagerstelle eines von dem Stechelement (10) abgewandten Schwenklagers (44) des Kniehebelmechanismus (22) oder der Blattfeder (84) zur Einstellung der Stechtiefe verstellbar ist.

18. Vorrichtung nach einem der Ansprüche 1 bis 17, **gekennzeichnet durch** ein insbesondere als Kolben-Zylinder-Einheit ausgebildetes Dämpfungselement (26) zur Verlangsamung der Rückbewegung des Stechelements (10).

19. Vorrichtung nach einem der Ansprüche 1 bis 18, **dadurch gekennzeichnet, dass** der Durchmesser des Aktuatordrahts (16,18) kleiner als 1 mm, vorzugsweise kleiner als 0,5 mm ist.

20. Vorrichtung nach einem der Ansprüche 1 bis 19, **dadurch gekennzeichnet, dass** die insbesondere als Nickel-Titan-Legierung ausgebildete Formgedächtnislegierung eine Umformungstemperatur von mehr als 100°C besitzt.

21. Vorrichtung nach einem der Ansprüche 1 bis 20, **dadurch gekennzeichnet, dass** der Aktuatordraht (16,18) mehrere über Umlenkmittel (36;100,88) geführte, nebeneinander verlaufende Drahtabschnitte aufweist.

22. Vorrichtung nach einem der Ansprüche 1 bis 21, **dadurch gekennzeichnet, dass** der Aktuatordraht (16,18) aus mehreren parallel zueinander verlaufenden Einzeldrähten besteht.

23. Vorrichtung nach einem der Ansprüche 1 bis 22, **dadurch gekennzeichnet, dass** die Vorwärtsbewegung des Stechelements (10) mindestens eine Größenordnung schneller als die Rückbewegung erfolgt.

## Claims

1. Device for obtaining body fluid for analytical purposes comprising a lancing element (10) that can be inserted into a body part and a drive (12) for a forward and backward lancing movement of the lancing element (10), **characterized in that** the drive (12) has at least one actuator wire (16, 18) based on shape memory alloys to control the lancing movement by means of a change in the wire length, wherein the change in length of the actuator wire (16, 18) is converted into a forward movement as well as into a directly subsequent return movement of the lancing element (10).

2. Device according to claim 1, **characterized in that** the actuator wire (16, 18) forms a propulsion means for the forward movement of the lancing element (10) by heat-activated contraction.

3. Device according to claim 1 or 2, **characterized in that** the drive (12) has a movement converter (22) arranged between the actuator wire (16, 18) and the lancing element (10) to convert a change in the length of the actuator wire (16, 18) into the lancing movement.

4. Device according to one of the claims 1 to 3, **characterized in that** the drive (12) comprises a heating unit (60) to heat up the actuator wire (16, 18) to a heating temperature which results in a contraction of the shape memory alloy.

5. Device according to one of the claims 1 to 4, **characterized in that** a current source, in particular a capacitor (64), which generates a current pulse is connected to the actuator wire (16, 18) via a trigger switch (62).

6. Device according to one of the claims 1 to 5, **characterized in that** the actuator wire (16, 18) can be heated over a variable length preferably by movable electrical contact points in order to adjust the lancing depth and/or collecting depth of the lancing element (10).

7. Device according to one of the claims 1 to 6, **characterized in that** the actuator wire (16, 18) is subjected to a partial contraction by specifically heating it to a transformation temperature in order to adjust the lancing depth and/or collecting depth of the lancing element (10).

8. Device according to one of the claims 1 to 7, **characterized in that** the drive (12) has a return means (24) that can be pretensioned during the forward movement of the lancing element (10) and in particular a return spring for the return movement of the lancing element (10).

9. Device according to one of the claims 1 to 8, **characterized in that** the actuator wire (16, 18) brakes and/or damps the return movement of the lancing element (10).

10. Device according to one of the claims 1 to 9, **characterized by** two alternately contractable actuator wires (16, 18) for alternately controlling the lancing movements in successive cycles.

11. Device according to claim 10, **characterized in that** one actuator wire (16) under contraction drives the forward movement of the lancing element (10) and the other actuator wire (18) under expansion brakes and/or damps the backward movement.

12. Device according to one of the claims 9 to 11, **characterized in that** the actuator wire (16, 18) acting as the braking means can be specifically shortened by preheating to a structural transformation temperature of the shape memory alloy and can be correspondingly elongated when it is cooled.

13. Device according to one of the claims 9 to 12, **characterized in that** the actuator wire (16, 18) which brakes or damps the return movement is preheated in sections.

14. Device according to one of the claims 9 to 13, **characterized in that** the actuator wire (16, 18) acts as a braking means and/or damping means by tension-induced phase transition of the shape memory alloy.

15. Device according to one of the claims 1 to 14, **characterized in that** the drive (12) comprises a knee lever mechanism (22) that can be stretched in the axis of the lancing movement or a correspondingly stretchable leaf spring (84).

16. Device according to claim 15, **characterized in that** the knee lever mechanism (22) or the leaf spring (84) can be brought into a stretched position by the actuator wire (16, 18) and into a deflected position by a return element (24) that is pretensioned in the stretched position.

17. Device according to claim 15 or 16, **characterized in that** the bearing position of a pivot bearing (44) of the knee lever mechanism (22) or of the leaf spring (84) that faces away from the lancing element (10) can be adjusted in order to adjust the lancing depth.

18. Device according to one of the claims 1 to 17, **characterized by** a damping element (26) in particular in the form of a piston-cylinder unit for slowing down the return movement of the lancing element (10).

19. Device according to one of the claims 1 to 18, **characterized in that** the diameter of the actuator wire (16, 18) is less than 1 mm, preferably less than 0.5 mm.

20. Device according to one of the claims 1 to 19, **characterized in that** the shape memory alloy which is in particular in the form of a nickel-titanium alloy has a transformation temperature of more than 100°C.

21. Device according to one of the claims 1 to 20, **characterized in that** the actuator wire (16, 18) has several wire sections guided side-by-side over deflection means (36; 100, 88).

22. Device according to one of the claims 1 to 21, **characterized in that** the actuator wire (16, 18) consists of several individual wires running parallel to one another.

23. Device according to one of the claims 1 to 22, **characterized in that** the forward movement of the lancing element (10) is at least an order of magnitude faster than the return movement.

## Revendications

1. Dispositif pour obtenir du liquide corporel pour des besoins d'analyse comprenant un élément de piquage (10) pouvant être piqué dans une partie du corps et un entraînement (12) pour un mouvement de piquage allant en avant et en arrière de l'élément de piquage (10), **caractérisé en ce que** l'entraînement (12) présente au moins un fil d'actionneur (16, 18) à base d'alliages à mémoire de forme pour la commande du mouvement de piquage en fonction d'une variation de la longueur du fil, la variation en longueur du fil d'actionneur (16, 18) étant convertie aussi bien en un mouvement en avant qu'en un mouvement en arrière, y faisant directement suite, de l'élément de piquage (10).

2. Dispositif selon la revendication 1, **caractérisé en ce que** le fil d'actionneur (16, 18) forme par contraction activée par chaleur un moyen d'avancement pour le mouvement en avant de l'élément de piquage (10).

3. Dispositif selon la revendication 1 ou 2, **caractérisé en ce que** l'entraînement (12) présente un convertisseur de mouvement (22) disposé entre le fil d'actionneur (16, 18) et l'élément de piquage (10) pour la conversion d'une variation de longueur du fil d'actionneur (16, 18) en le mouvement de piquage.

4. Dispositif selon l'une quelconque des revendications 1 à 3, **caractérisé en ce que** l'entraînement (12) présente une unité de chauffage (60) pour le chauffage du fil d'actionneur (16, 18) jusqu'à une température de chauffage entraînant la contraction de l'alliage à mémoire de forme.

5. Dispositif selon l'une quelconque des revendications 1 à 4, **caractérisé en ce qu'**une source de courant générant une impulsion électrique, en particulier un condensateur (64), est raccordé au fil d'actionneur (16, 18) au moyen d'un interrupteur de déclenchement (62).

6. Dispositif selon l'une quelconque des revendications 1 à 5, **caractérisé en ce que** le fil d'actionneur (16, 18) peut être chauffé pour le réglage de la profondeur de piquage et/ou de la profondeur de collecte de l'élément de piquage (10) de préférence par des points de contact électriques déplaçables sur une longueur variable.

7. Dispositif selon l'une quelconque des revendications 1 à 6, **caractérisé en ce que** le fil d'actionneur (16, 18) est soumis à une contraction partielle pour le réglage de la profondeur de piquage et la profondeur de collecte de l'élément de piquage (10) par réchauffement ciblé jusqu'à une température de déformation.

8. Dispositif selon l'une quelconque des revendications 1 à 7, **caractérisé en ce que** l'entraînement (12) présente un moyen de rappel (24) pouvant être prétendu lors du mouvement en avant de l'élément de piquage (10), en particulier un ressort de rappel pour le mouvement en arrière de l'élément de piquage (10).

9. Dispositif selon l'une quelconque des revendications 1 à 8, **caractérisé en ce que** le fil d'actionneur (16, 18) ralentit et/ou amortit le mouvement en arrière de l'élément de piquage (10).

10. Dispositif selon l'une quelconque des revendications 1 à 9, **caractérisé par** deux fils d'actionneur (16, 18) contractables alternativement pour la commande alternante des mouvements de piquage dans des cycles consécutifs.

11. Dispositif selon la revendication 10, **caractérisé en ce qu'**un fil d'actionneur (16) entraîne sous contraction le mouvement en avant de l'élément de piquage (10) et l'autre fil d'actionneur (18) ralentit et/ou amortit le mouvement en arrière sous expansion.

12. Dispositif selon l'une quelconque des revendications 9 à 11, **caractérisé en ce que** le fil d'actionneur (16, 18) agissant comme moyen de freinage est raccourci de façon ciblée par préchauffage jusqu'à une température de déformation de structure de l'alliage à mémoire de forme et peut être allongé en conséquence par refroidissement.

13. Dispositif selon l'une quelconque des revendications 9 à 12, **caractérisé en ce que** le fil d'actionneur (16, 18) ralentissant ou amortissant le mouvement de recul est préchauffé par portions.

14. Dispositif selon l'une quelconque des revendications 9 à 13, **caractérisé en ce que** le fil d'actionneur (16, 18) agit comme moyen de freinage et/ou moyen d'amortissement par conversion de phase, induite par tension de traction, de l'alliage à mémoire de forme.

15. Dispositif selon l'une quelconque des revendications 1 à 14, **caractérisé en ce que** l'entraînement (12) comprend un mécanisme à levier coudé (22) étirable dans l'axe du mouvement de piquage ou un ressort à lame (84) étirable de façon appropriée.

16. Dispositif selon la revendication 15, **caractérisé en ce que** le mécanisme à levier coudé (22) ou le ressort à lame (84) peut être amené dans une position d'étirage par le fil d'actionneur (16, 18) et dans une position de flexion par un élément de rappel (24) prétendu dans la position d'étirage.

17. Dispositif selon la revendication 15 ou 16, **caractérisé en ce que** le point de palier d'un palier basculant (44), opposé à l'élément de piquage (10), du mécanise à levier coudé (22) ou du ressort à lame (84) est réglable pour le réglage de la profondeur de piquage.

18. Dispositif selon l'une des revendications 1 à 17, **caractérisé par** un élément d'amortissement (26) conçu en particulier comme unité piston-cylindre pour le ralentissement du mouvement en arrière de l'élément de piquage (10).

19. Dispositif selon l'une quelconque des revendications 1 à 18, **caractérisé en ce que** le diamètre du fil d'actionneur (16, 18) est inférieur à 1 mm, de préférence inférieur à 0,5 mm.

20. Dispositif selon l'une quelconque des revendications 1 à 19, **caractérisé en ce que** l'alliage à mémoire de forme, conçu en particulier sous forme d'alliage nickel-titane, présente une température de déformation supérieure à 100°C.

21. Dispositif selon l'une quelconque des revendications 1 à 20, **caractérisé en ce que** le fil d'actionneur (16, 18) présente plusieurs portions de fil guidés par des moyens de déviation (36 ; 100, 88) et agencés les uns à côté des autres.

22. Dispositif selon l'une quelconque des revendications 1 à 21, **caractérisé en ce que** le fil d'actionneur (16, 18) est constitué de plusieurs fils individuels agencés parallèlement entre eux.

23. Dispositif selon l'une quelconque des revendications 1 à 22, **caractérisé en ce que** le mouvement en avant de l'élément de piquage (10) s'effectue plus rapidement, d'au moins un ordre de grandeur, que le mouvement en arrière.
